# EUROPEAN PATENT SPECIFICATION

(11) **EP 0 472 423 B1**
(45) Date of publication and mention of the grant of the patent: **03.07.1996**
(21) Application number: 91307704.6
(22) Date of filing: 21.08.1991
(51) Int. Cl.: C08G 65/32, C07C 41/01, C07C 43/12, C07C 51/58, C08G 65/00, C07C 41/00

(54) **Preparation of perfluorooligoether iodides**
Herstellung von Perfluorooligoetheriodiden
Préparation d'iodides de perfluorooligoéthers

(30) Priority: 23.08.1990 JP 222090/90
(43) Date of publication of application: 26.02.1992
(73) Proprietor: SHIN-ETSU CHEMICAL CO., LTD., Chiyoda-ku Tokyo (JP)
(72) Inventor: Oyama, Masayuki, Gunma-gun, Gunma-ken (JP); Koike, Noriyuki, Tano-gun, Gunma-ken (JP); Takago, Toshio, Annaka-shi, Gunma-ken (JP)
(74) Representative: Stoner, Gerard Patrick

(56) References cited:
- EP-A- 0 348 948
- DATABASE WPIL Week 8811, Derwent Publications Ltd., London, GB; AN 88-075284
- THE JOURNAL OF ORGANIC CHEMISTRY vol. 30, no. 7, July 1965, pages 2182 - 2190 J. F. HARRIS 'The Photolysis of Polyfluoroacyl Fluorides, Chlorides, and Bromides'

## Description

This invention relates to new methods for preparing perfluorooligoether iodides, which are useful intermediates for e.g. synthesis of fluoro resins, fluoro rubbers, and fluoro surfactants.

### BACKGROUND

In the past, methods for preparing perfluoroalkyl iodides by telomerization of tetrafluoroethylene with trifluoromethyl iodide, or by derivation from alcohols, have been used in commercial practice.

However, no commercially acceptable methods have been developed for the preparation of perfluorooligoether iodides of the following general formula ( II ): 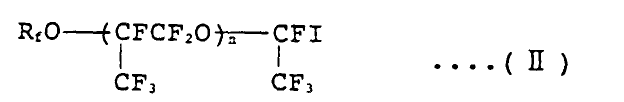 wherein Rf is a perfluoroalkyl group having 1 to 10 carbon atoms and letter n is an integer of from 0 to 100, which are useful intermediates for the synthesis of fluoro resins, fluoro rubbers, and fluoro surfactants.

In the prior art, perfluorooligoether iodides have been made (as shown by the following reaction scheme) by starting with a perfluorooligoether carboxylic fluoride of the following formula ( III ), subjecting the reactant to hydrolysis to form a perfluorooligoether carboxylic acid, reacting it with silver oxide to form a perfluorooligoether carboxylic silver salt, and subjecting the salt to pyrolysis in the presence of iodine to thereby form a perfluorooligoether iodide of formula (II). See JP-A-30441/1988.

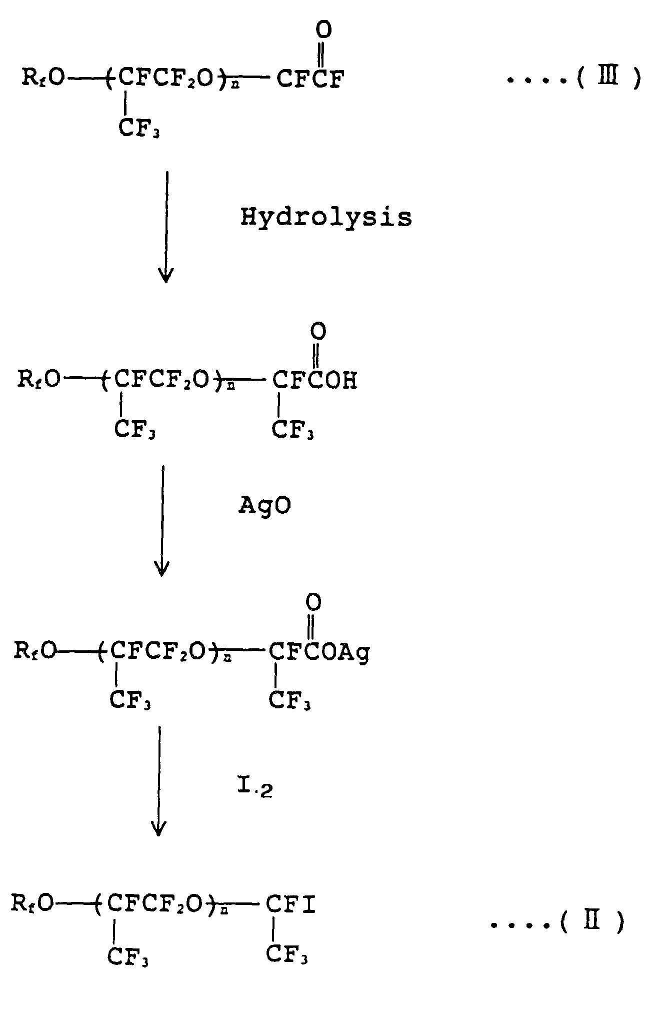

However, several problems must be solved before this process can be commercially practised. The end product, perfluorooligoether iodide, is recovered only in yields of about 70 to 85%. The intermediate or silver salt is often a solid which is difficult to handle. The use of expensive silver necessitates silver recovery from a commercial standpoint. The overall process involves three steps, during which toxic hydrogen fluoride evolves. Pyrolysis reaction must be carried out before the end product can be obtained. Also, economical problems arise from the use of expensive reactants and increased installation cost.

There is a need for the commercially advantageous manufacture of perfluorooligoether iodides while overcoming the above - mentioned problems.

As claimed in our EP-A-047 2422 with the same priority date, we have found that by effecting halogen exchange reaction between a perfluorooligoether carboxylic fluoride of formula (III): 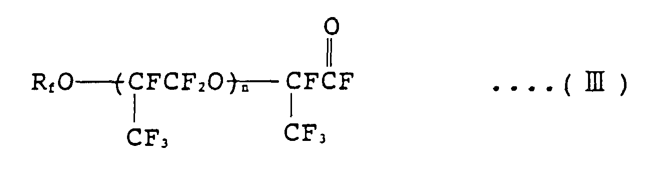 wherein Rf and n as defined above and a metal iodide of the general formula (IV):

MIₐ (IV)

wherein M is a metal atom and letter a is the valence of the metal atom, there is obtained a novel perfluorooligoether carboxylic iodide of the general formula (I): 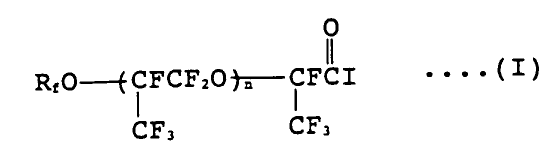 wherein Rf and n are as defined above. We have also found that by exposing a perfluorooligoether carboxylic iodide of formula (I) to ultraviolet light, carbon monoxide removal readily takes place to produce an end product, perfluorooligoether iodide of formula ( II ): 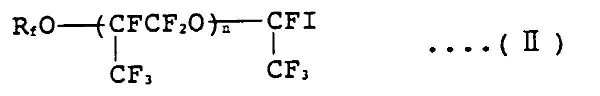 wherein Rf and n are as defined above, and that it may be obtained in high yields.

More particularly, since the reaction between the compounds of formulae ( III ) and ( IV ) may proceed in a simple manner even at atmospheric pressure and room temperature without forming a by-product, a novel perfluorooligoether carboxylic iodide of formula (I) is obtainable in high yields. This novel perfluorooligoether carboxylic iodide releases carbon monoxide upon exposure to ultraviolet light at atmospheric pressure and room temperature, and may convert to end product, perfluorooligoether iodide of formula ( II ), at a conversion rate of 95% or higher without forming a by-product. The step of ultraviolet exposure may involve only irradiation with ultraviolet light; no other special operation is needed. The reaction system may thus be raised to any desired scale in theory insofar as a tank for receiving the reaction solution is installed in the system.

As compared with the prior art process, perfluorooligoether iodide preparing methods embodying the invention offer possible advantages.
(1) Very high yield.
(2) No use of expensive silver oxide.
(3) A simplified process.
(4) No evolution of toxic hydrogen fluoride during the process.
(5) Low reaction temperature.
(6) The intermediate or perfluorooligoether carboxylic iodide of formula (I) is liquid at room temperature and can be isolated by distillation if it has a low molecular weight.

Therefore, the present method allows a perfluorooligoether iodide to be prepared with many commercial benefits.

In a first specific aspect, the invention provides a method for preparing a perfluorooligoether iodide of the general formula (II): 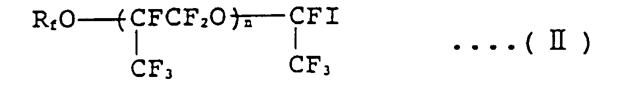 wherein Rf is a perfluoroalkyl group having 1 to 10 carbon atoms, and n is an integer of from 0 to 100, said method comprising the step of exposing to ultraviolet light a perfluorooligoether carboxylic iodide of the general formula (I): 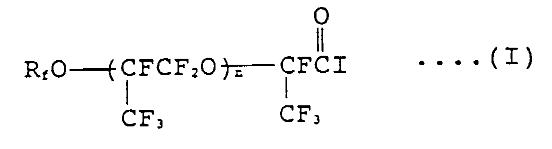 wherein Rf and n are as defined above.

In a second aspect, the invention provides an overall process for preparing a perfluorooligoether iodide of formula (II), comprising preparing a perfluorooligoether carboxylic iodide of formula (I) and then treating the iodide with UV radiation to convert it to the iodide of formula (II).

In a third aspect, the invention provides use of a perfluorooligoether carboxylic iodide of formula (I) as an intermediate in the preparation of a compound of formula (II), to give an improved yield.

### BRIEF DESCRIPTION OF THE DRAWINGS

FIG. 1 is a chart showing the IR spectrum of a perfluorooligoether carboxylic iodide obtained in an Example.

FIG. 2 is a chart showing the IR spectrum of a perfluorooligoether iodide obtained in an Example.

The present invention is intended to prepare a perfluorooligoether iodide of formula ( II ) by exposing a perfluorooligoether carboxylic iodide of formula (I) to ultraviolet light. The starting reactant, that is, perfluorooligoether carboxylic iodide of formula (I) is obtained by effecting halogen exchange reaction between a perfluorooligoether carboxylic fluoride and a metal iodide.

The perfluorooligoether carboxylic fluorides are compounds of the general formula ( III ):

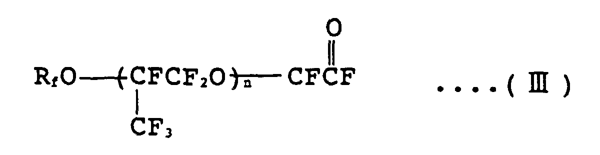

In formula (III), Rf is a perfluoroalkyl group having 1 to 10 carbon atoms, for example, trifluoromethyl, pentafluoroethyl, heptafluoropropyl, and heptafluoroisopropyl groups. Letter n is an integer of from 0 to 100, preferably from 0 to 30. The perfluorooligoether carboxylic fluorides of formula (III) may be prepared by any conventional well-known methods, e.g. as disclosed in US-A-3,250,808 and US-A-3,322,826.

With the perfluorooligoether carboxylic fluorides of formula (III) are reacted metal iodides of the general formula (IV):

MIₐ (IV)

wherein M is a metal atom and letter a is the valence of the metal atom. Suitable iodides include alkali metal iodides such as LiI and NaI, alkaline earth metal iodides such as MgI₂ and CaI₂, and other metal iodides such as AlI₃. The metal iodide is preferably used in such amounts that the molar amount of iodine in the metal iodide is 1 to 1.2 times the moles of the perfluorooligoether carboxylic fluoride of formula ( III ). For example, the alkali metal iodide is 1 to 1.2 mol and the alkaline earth metal iodide is 0.5 to 0.6 mol per mol of the formula ( III ) compound.

These reactants are reacted, for example, by adding a polar solvent to the perfluorooligoether carboxylic fluoride of formula ( III ) and with stirring, adding thereto the metal iodide in increments. The preferred polar solvents used herein are aprotic solvents such as diethyl ether, diisopropyl ether, dibutyl ether, and acetonitrile. The solvents are preferably used in amounts of about 2 to 10% by weight of the alkali metal iodide or about 4 to 20% by weight of the alkaline earth metal iodide. The reaction temperature generally ranges from 0°C to 100°C, preferably 20°C to 50°C and the reaction time generally ranges from about 2 to about 50 hours, preferably from about 5 to about 10 hours. Since both the reactants and the product are prone to hydrolysis, it is recommended to purge the reactor thoroughly with an inert gas such as nitrogen and argon. At the end of reaction, the metal fluoride is removed by filtration and then, the perfluorooligoether carboxylic iodide of formula (I): 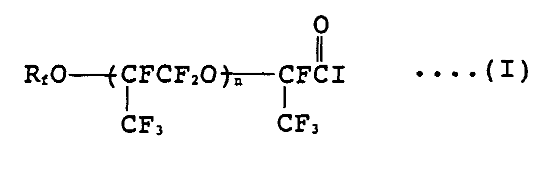 wherein Rf and n are as defined above can be recovered from the reaction solution in high yields by distillation isolation or by distilling off the solvent.

The resultant perfluorooligoether carboxylic iodide of formula (I) can be readily converted into an end product, perfluorooligoether iodide of formula ( II ) in high yields simply by exposing the former to UV light, causing it to release carbon monoxide.

For UV exposure, a UV irradiation apparatus having a high pressure mercury lamp with a cooling quartz jacket may be used. Reaction is conducted by exposing the charge in the photo-reactor to UV at a wavelength of 180 to 380 nm, preferably 200 to 300 nm at a temperature of 0 to 60°C, preferably room temperature for about 2 to about 30 hours. There is no need for solvent although the charge may be diluted with a stable organic solvent if desired. Such solvents are perfluorooctane, perfluoroisononane and the like. Also preferably, this reaction is effected in an inert gas atmosphere such as nitrogen and argon.

With respect to the photo-reaction of perfluoroalkyl carboxylic halides, reference is made to Hassow, J. Org. Chm., 30, 2182 (1965), reporting the synthesis of RfBr in high yields by UV exposure of perfluorocarboxylic bromide RfCOBr.

The perfluorooligoether iodides of formula (II): 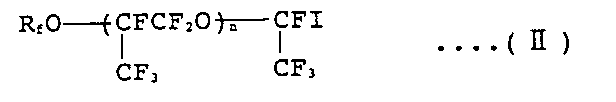 which are obtained in this way are useful intermediates e.g. for synthesis of fluoro resins, fluoro rubbers and fluoro surfactants.

There has been described a commercially advantageous method for preparing perfluorooligoether iodides of formula ( II ) in high yields which are useful intermediates for e.g. synthesis of fluoro resins, fluoro rubbers and fluoro surfactants.

### EXAMPLE

Examples of the present invention are given below by way of illustration and not by way of limitation.

### Example

A 1/2 - liter four-necked flask equipped with a mechanical stirrer, reflux condenser, and gas inlet tube was charged with 400 grams (0.80 mol) of a perfluorooligoether carboxylic fluoride of the formula: 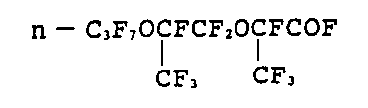 and 8 grams acetonitrile. While stirring the contents, 118 grams (0.88 mol) of lithium iodide was added to the flask in several portions in an argon stream. The lithium iodide was added at a controlled rate such that the temperature of the contents did not exceed 40°C. At the end of addition, the contents were stirred for a further 15 hours.

The contents were passed through a glass filter to remove the solids. Distillation of the filtrate yielded 421 grams of a fraction having a boiling point of 87-90°C/106,7 mbar (80 mmHg). The yield was 87%.

The product was analyzed by elemental analysis, GC - MS, IR spectroscopy, and ¹⁹F-NMR. The results are shown below.

| Elemental analysis | | | | |
|---|---|---|---|---|
| | C | F | I | O |
| Calcd., % | 17.82 | 53.30 | 20.96 | 7.92 |
| Found, % | 17.79 | 53.37 | 20.91 | 7.93 |

### GC-MS

m/e (M⁺) molecular weight 606

### IR spectroscopy

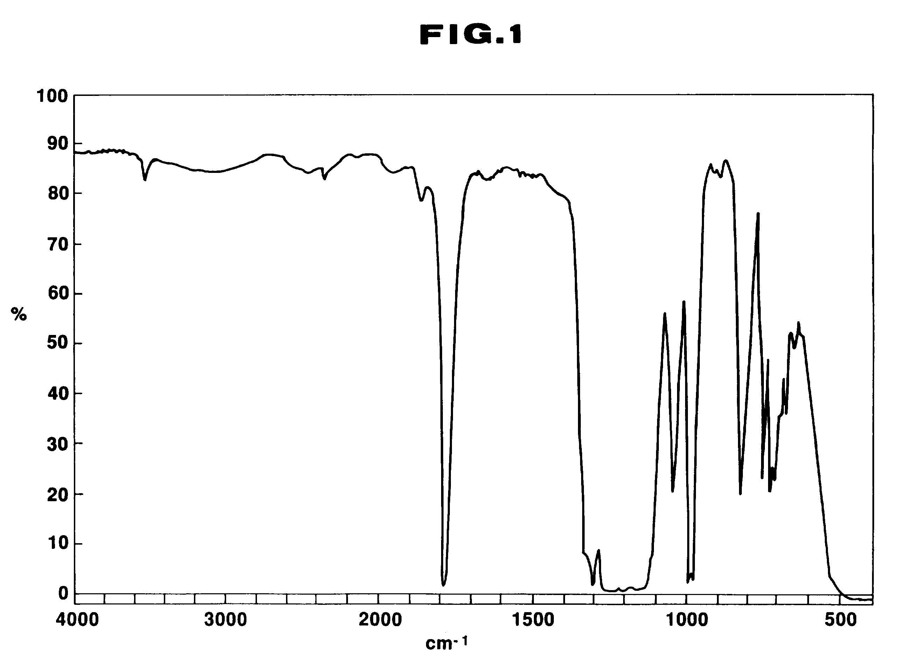

FIG. 1 is a chart showing the IR spectrum of the product. It is observed that the absorption peak at 1890 cm⁻¹ attributable to -CO-F disappeared and a new peak developed at 1785 cm⁻¹ attributable to -CO-I.

### ¹⁹F-NMR

- δ (ppm):: 66.5 (m, 1F, CF)
- 52.8 (m, 2F, CF₂)
- 41.0 (m, 1F, CF - COI)
- - 1.5 - 7.1 (m, 13F, - CF₃, CF₂O - )

With these measurement results, the product was identified to be a perfluorooligoether carboxylic iodide of the following formula.

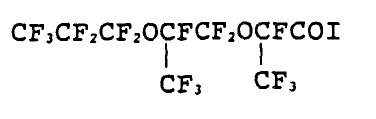

Next, a UV irradiation apparatus was equipped with a high pressure mercury lamp with a cooling quartz jacket. The apparatus was charged with 200 grams (0.33 mol) of the perfluorooligoether carboxylic iodide, which was exposed to UV light while stirring with a magnetic stirrer. The lamp was operated at a power of 100 W and a wavelength of 220 to 380 nm. Reaction was continued for 16 hours at 35 to 40°C in an argon stream. At the end of reaction, the reaction product was distilled, obtaining 181 grams of a fraction having a boiling point of 78.5°C 134,7 mbar (101 mmHg). The yield was 95%.

The product was analyzed by elemental analysis, GC - MS, IR spectroscopy, and ¹⁹F - NMR. The results are shown below.

| Elemental analysis | | | | |
|---|---|---|---|---|
| | C | F | I | O |
| Calcd., % | 16.61 | 55.88 | 21.79 | 5.54 |
| Found, % | 16.57 | 55.91 | 21.92 | 5.60 |

### GC - MS

m/e (M⁺) molecular weight 578

### IR spectroscopy

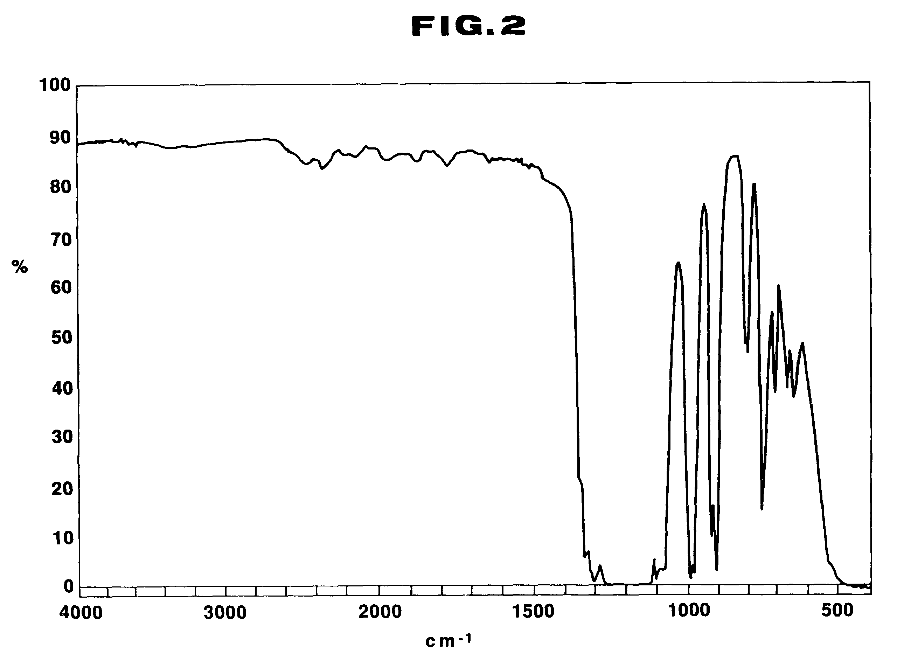

FIG. 2 is a chart showing the IR spectrum of the product. It is observed that the absorption peak at 1785 cm⁻¹ attributable to - CO - I disappeared.

### ¹⁹F - NMR

- δ (ppm):: 69.9 (m, 1F, CF)
- 59.8 (m, 2F, CF₂)
- 3.7 - 15.8 (m, 13F,
- - CF₃, - CF₂O - )
- - 0.3 (m, 1F, -CFI)

With these measurement results, the product was identified to be a compound of the following formula.

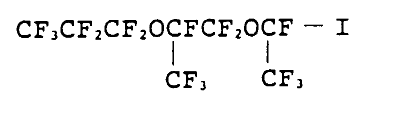

## Claims

1. A method for preparing a perfluorooligoether iodide of the general formula ( II ): 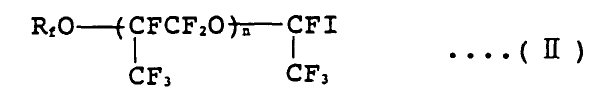 wherein R_{f} is a perfluoroalkyl group having 1 to 10 carbon atoms, and n is an integer from 0 to 100, said method comprising the step of exposing to ultraviolet light a perfluorooligoether carboxylic iodide of the general formula (I): 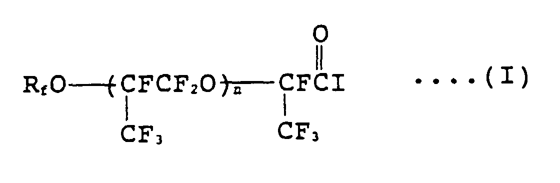 wherein R_{f} and n are as defined above.

## Patentansprüche

1. verfahren zur Herstellung eines Perfluoroligoetherjodids der allgemeinen Formel (II): 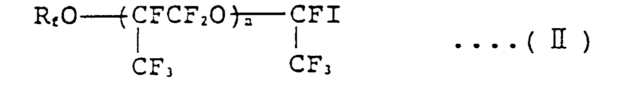 worin R_{f} eine Perfluoralkylgruppe mit 1 bis 10 Kohlenstoffatomen ist und n eine ganze Zahl von 0 bis 100 ist, wobei das Verfahren den Schritt des Bestrahlens eines Perfluoroligoethercarboxyljodids der allgemeinen Formel (1) 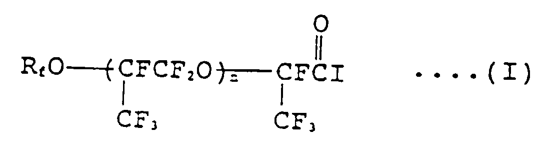 worin Rᵢ und n die oben angeführte Bedeutung haben, mit UV-Licht umfaßt.

## Revendications

1. Méthode de préparation d'un perfluorooligoéther iodure de formule générale (II) : 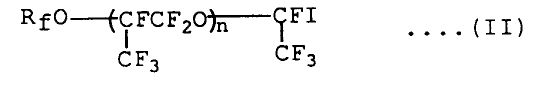 où R_{f} est un groupe perfluorooalkyle ayant de 1 à 10 atomes de carbone, et n est un entier valant de 0 à 100, ladite méthode comprenant l'étape d'exposition à une lumière ultraviolette d'un iodure perfluorooligoéther carboxylique de formule générale (I) : 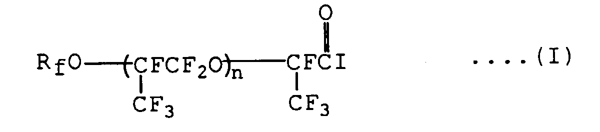 où R_{f} et n sont tels que définis ci-dessus.
